(19) Europäisches Patentamt
European Patent Office
Office européen des brevets

(11) **EP 1 690 525 B1**

(12) **EUROPEAN PATENT SPECIFICATION**

(45) Date of publication and mention
of the grant of the patent:
**02.11.2016 Bulletin 2016/44**

(51) Int Cl.:
*A61K 8/44* *(2006.01)*      *A61K 8/88* *(2006.01)*
*A61K 8/04* *(2006.01)*      *A61Q 19/00* *(2006.01)*
*A61Q 19/02* *(2006.01)*

(21) Application number: **05250114.5**

(22) Date of filing: **12.01.2005**

(54) **Gamma-polyglutamate hydrogels for use as super moisturizers in cosmetic and personal care products**

Gamma-Polyglutamat Hydrogele zur Anwendung als Feuchthaltemittel in der Kosmetik und bei der persönlichen Pflege

Hydrogels de gamma-polyglutymate utilisés comme humectants dans des produits cosmetiques et de soin personnel

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**16.08.2006 Bulletin 2006/33**

(73) Proprietor: **Tung Hai Biotechnology Corporation Taichung Hsien (TW)**

(72) Inventors:
• **Ho, Guan-Huei**
**Mississauga, Ontario L5N 7Y6 (CA)**
• **Yang, Jeng**
**Taichung (TW)**

• **Yang, Tou-Hsiung**
**Taichung (TW)**

(74) Representative: **Sexton, Jane Helen**
**J A Kemp**
**14 South Square**
**Gray's Inn**
**London WC1R 5JJ (GB)**

(56) References cited:
**EP-A1- 1 550 469**      **WO-A-2004/007593**
**WO-A-2004/039339**      **WO-A-2004/080433**
**JP-A- H11 343 339**      **JP-A- 2001 354 542**
**JP-A- 2002 145 723**      **JP-A- 2003 146 830**
**JP-A- 2004 210 699**      **US-B2- 7 364 879**

EP 1 690 525 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD OF THE INVENTION**

This invention relates to use of cross-linked $\gamma$-polyglutamate hydrogels (prepared from $\gamma$-polyglutamate in $Na^+$ form, $\gamma$-polyglutamate in $K^+$ form, $\gamma$-polyglutamate in $NH_4^+$ form, $\gamma$-polyglutamate in $Mg^{++}$ form and/or $\gamma$-polyglutamate in $Ca^{++}$ form) in cosmetic and personal care products to provide desired functionalities including high moisturization and water retention, improved wetability and low TEWL (trans-epidermal water loss), soft and tender feel, smooth and dryness, long lasting effectiveness, enhancing skin elasticity, good biocompatibility and enhancing health condition of the skin.

**TECHNICAL BACKGROUND AND PRIOR ART**

Proper moisturization and nutrition are vital to the health and beauty of human skin and hair. Over dryness caused by low humidity is often detrimental to the skin and hair conditions. In winter, low temperature and dry air especially cause the dryness of the skin and hair, deteriorating the skin health conditions and even hardening or damaging the epidermis and electrifying the hair. To prevent the dryness of the skin, hair, and nail, cosmetic products such as skin essence, hand and body lotions, bath soaps, skin and body creams, hair gels, hair shampoos and mousse, and many other personal care products often contain certain moisturizers to provide the necessary moisturizing conditions to the skin and hair, and also to protect and beautify the skin and hair.

There are many kinds of organic moisturizers being used in a variety of cosmetic and toiletry products on the market. But the water absorption capacity, safety requirement of the moisturizers and the long stability concerns largely limit the kinds of moisturizers used in practical applications. A good moisturizer must possess high water retention capacity and also be capable of decreasing water loss through evaporation from the skin and hair. Traditional moisturizers being used in the cosmetics industry include glycerin, di-glycerol, sorbitol, sodium lactate, propylene glycol, and amino acid. Among them, sodium lactate has better water retention capacity, but is difficult to be emulsified in the formulation of the end products, therefore is only found limited applications and its usage level is low. The polyols have better moisturizing effect but have little effectiveness in the cosmetic products. Hyaluronic acid, collagen and squalane possess good water retention capacity, but they are less effective in reducing water evaporation from the surface of skin, and become sticky on the skin surface. Besides those above-mentioned moisturizers, elastin, glucosamine, polyaspartic acid (see JP 61-033107), placenta, chondroitin, aloe vera extract, and amino acid esters (see JP 10-251402) are also being used as moisturizing ingredients in the cosmetic and personal care formulations. The search for better and improved moisturizer is continued worldwide.

The polyols such as 1,3-butyl glycol, ethylene glycol, propylene glycol, and polyethylene glycol possess certain recognized moisturization capacity, inhibit the growth of some microbes, improve the miscibility and viscosity, and provide some stability to other ingredients used in the cosmetic and personal care products, including tonic essence, skin cream, skin and body lotion, jelly, hair shampoo, conditioners, anti-drying treatments, hair tonic, bath and moisturizing creams etc. Despite those benefits from the polyols, the skin-care and hair-care cosmetic products containing the above-mentioned polyols always leave a not-so-good feeling to the skin, or slightly dry feeling to the hair, and make consumer feel the results are not better than those cosmetic products containing no polyols. More often, those rinse and cleansing cosmetics such as skin lotions, face creams, and hair setting, always leave residual polyols in the hair or on the skin, and make the user sometimes feel bad, decreasing their desire to continue to use.

Hyaluronic acid (HA) has relatively good water absorption and retention capability. HA is a natural biopolymer, non-toxic and fully biocompatible with human body, and is being used in most high quality cosmetic products for its effectiveness in preventing dryness of skin and hair. The disadvantage of using hyaluronic acid is its high price and limited availability. Despite its excellent moisturization functionality in protecting skin from over drying condition, the extremely high price of HA will result in a higher cost of the cosmetic formulation of the end products. Recent BSE virus protein-prion scare and Asian avian fluenza spreading have triggered serious concern about the safety of HA. Though, squalane has the advantage of moistening skin surface by preventing water from being evaporated from skin surface, its oily nature makes the user feel greasy on skin. The commercial collagens are extracted from animal sources, implicating a high risk of contaminating BSE protein prion and possibility of infection with wide spread avian fluenza virus or impurity.

WO 2004007593 relates to a poly-$\gamma$-glutamate (PGA) having n ultra-high molecular weight greater than 5000 kDa which has moisture-absorbing, moisture retaining, sustained release, mineral solubility, and water-absorbing properties. WO 2004080433 provides a hair treatment composition containing $\gamma$-polyglutamic acid or its salt, a hair cosmetic for damaged hair containing the same, and use thereof. WO-A-2004039339 discloses a cosmetic which contains crosslinked poly-$\gamma$-glutamic acid compound and/or crosslinked poly-$\gamma$-glutaric acid salt having a particle size of from 0-1 to 100 $\mu$m and an average particle size of from 1 to 50 $\mu$m. JP2003-146830 provides a cosmetic comprising a radiation-crosslinked body of a polyamino acid or a polyamino acid salt. JP2004-210699 provides a cosmetic composition containing a γ-polyglutamic acid having a high thickening effect and a humectant effect, and further having greater than 2,000,000 average molecular weight or salt thereof, especially the γ-polyglutamic acid having greater than 3,000,000 average molecular weight or a salt thereof. On a different note, JP 11 343339 A describes biodegradable resins made from poly-γ-glutamic acid and ethylene-glycol-diglycidyl-ether.

## CONTENT OF THE INVENTION

In this invention, use of certain cross-linked γ-polyglutamate hydrogels prepared from γ-polyglutamate in $Na^+$ form, γ-polyglutamate in $K^+$ form, γ-polyglutamate in $NH_4^+$ form, γ-polyglutamate in $Mg^{++}$ form, γ-polyglutamate in $Ca^{++}$ form, or a mixture of two or more thereof, as a moisturizer, which has excellent moisturizing effect on skin and hair at reasonable cost, adds health values to the cosmetic and personal care products.

## DESCRIPTION OF THE DRAWINGS

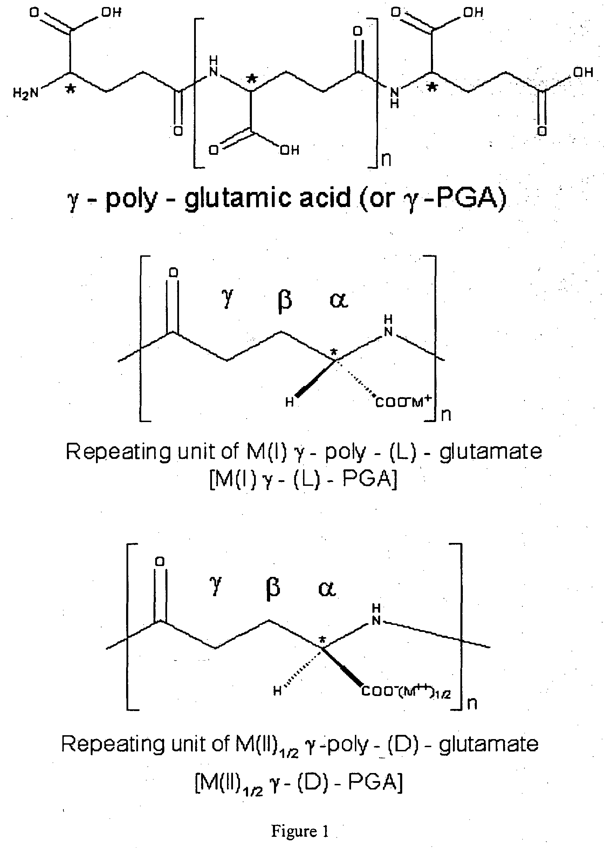

Figure 1 shows the chemical structure of γ-polyglutamic acid (γ-PGA, H form) (A), γ-polyglutamate in $K^+$ form, γ-polyglutamate in $Na^+$ form, and γ-polyglutamate in $NH_4^+$ form (B), and γ-polyglutamate in $Ca^{++}$ form arid γ-polyglutamate in $Mg^{++}$ form (C). M(I)=$K^+$, $Na^+$, or $NH_4^+$ M(II)=$Ca^{++}$ or $Mg^{++}$

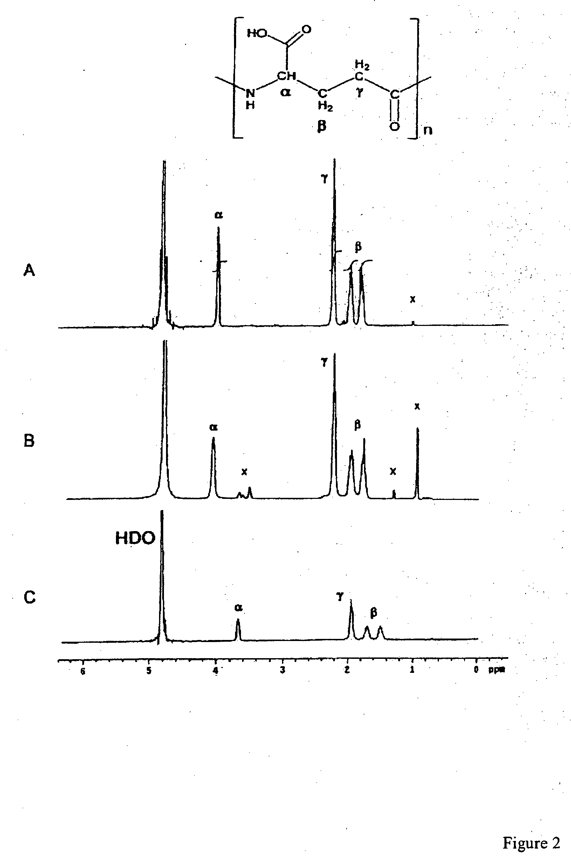

Figure 2 shows 400 MHz $^1$H-NMR spectra of γ-polyglutamate in $Na^+$ form (A), γ-polyglutamate in $K^+$ form (B), and γ-polyglutamate in $NH_4^+$ form (C) in $D_2O$ at neutral pH and temperature of 30°C. Chemical shift was measured in ppm units from the internal standard. X indicates impurity peak.

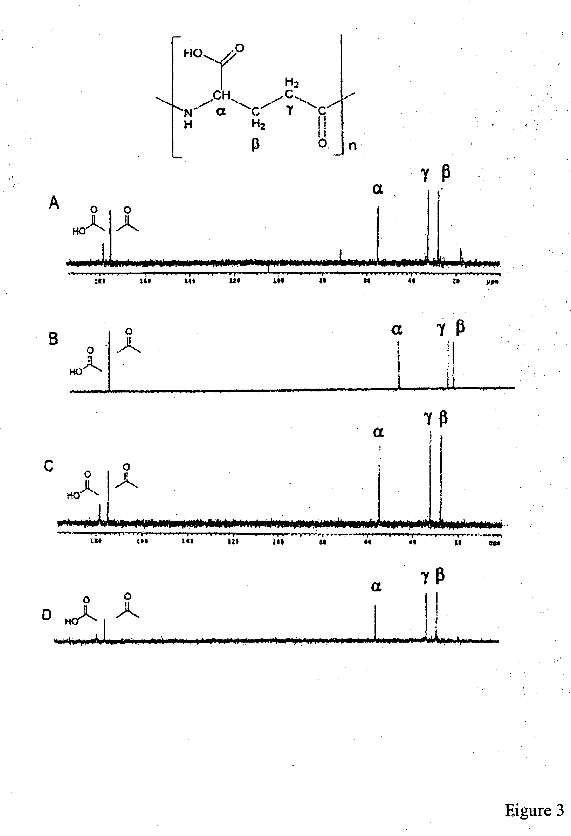

Figure 3 shows $^{13}$C-NMR spectra of γ-polyglutamate in $K^+$ form (A), γ-polyglutamate in $Na^+$ form (B), γ-polyglutamate in $Ca^{++}$ form (C), and γ-polyglutamate in $Mg^{++}$ form (D) in $D_2O$ at neutral pH and temperature of 30°C: Chemical shift was measured in ppm units from the internal reference.

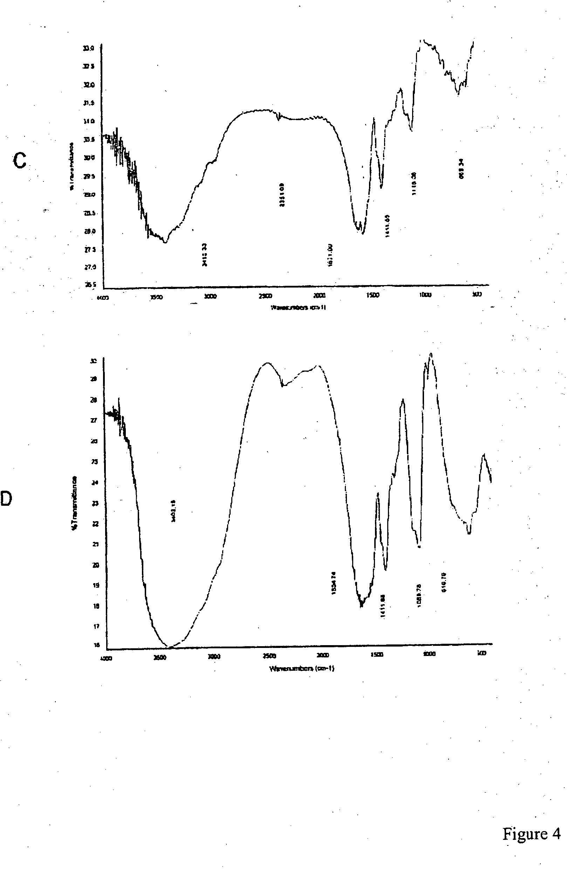

Figure 4 shows infrared (FT-IR) absorption spectra of γ-polyglutamate in $Ca^{++}$ form (C) and γ-polyglutamate in $Mg^{++}$ form (D) in KBr pellet.

## DETAILED DESCRIPTION OF THE INVENTION

The inventors, after thorough investigation, have come up with an excellent moisturizer by using of biopolymers prepared from salts of γ-polyglutamic acid (γ-PGA, H form) i.e., γ-polyglutamate in $Na^+$ form, γ-polyglutamate in $K^+$ form, γ-polyglutamate in $NH_4^+$ form, γ-polyglutamate in $Ca^{++}$ form, and γ-polyglutamate in $Mg^{++}$ form, which are all natural, biodegradable, non-toxic, and fully biocompatible. The typical chemical structures of γ-polyglutamic acid (γ-PGA, H form) and its salts are shown in Figure 1, and the typical $^1$H-NMR, $^{13}$C- NMR, and FT-IR are shown in Figures 2, 3, and 4, respectively. Table 1 shows the summarized data of chemical shifts of $^1$H-NMR, $^{13}$C-NMR, FT-IR absorption peaks, and the thermal analysis. The products containing the above biopolymers possess excellent water absorption capacity and long-lasting good water retention, form soft tender smooth thin film with matrix structure for controlled release function for water and other nutrients in the water, improve the elasticity of the skin, reduce the wrinkles by anti-radical activity in aging process, beautify and improve the health conditions of skin by increasing the natural moisturing factors (NMF) (see JP 2002-145723) in the stratum corneum, and reduce transepidermal water loss (TEWL) from the epidermis.

Table 1

| ITEM | H | $Na^+$ | $K^+$ | $NH_4^+$ | $Ca^{++}$ | $Mg^{++}$ |
|---|---|---|---|---|---|---|
| a. $^1$H-NMR(400MHz, $D_2O$, 30°C)<br>Chemical shift in ppm:<br>  α CH<br>  β $CH_2$<br>  γ $CH_2$ | | 3.98<br>1.98, 1.80<br>2.19 | 4.00<br>1.99, 1.80<br>2.19 | 3.68<br>1.68, 1.48<br>1.93 | 4.18<br>2.16, 1.93<br>2.38 | 4.08<br>2.05,<br>1.88<br>2.31 |
| b. $^{13}$C-NMR (67.9MHz, $D_2O$, 30°C)<br>Chemical shift in ppm; | | | | | | |

(continued)

| ITEM | H | Na+ | K+ | NH4+ | Ca++ | Mg++ |
|---|---|---|---|---|---|---|
| $\alpha$ CH | | 56.43 | 62.21 | | 62.21 | 62.10 |
| $\beta CH_2$ | | 31.61 | 35.16 | | 36.17 | 35.11 |
| $\gamma$ CH$_2$ | | 34.01 | 39.74 | | 39.68 | 39.60 |
| CO | | 182.21 | 182.11 | | 182.16 | 182.12 |
| COO$^-$ | | 182.69 | 185.46 | | 185.82 | 185.16 |
| a. FT-IR absorption (KBr), cm$^{-1}$ | | | | | | |
| C=O, Stretch | 1739 | | | | | |
| Amid I, N-H bending | | 1643 | | 1643 | 1622 | 1654 |
| Amide II, stretch | | 1585 | | | | |
| C=O, symmetric stretch | 1454 | 1402 | | 1395 | 1412 | 1411 |
| C-N, stretch | 1162 | 1131 | | 1139 | 1116 | 1089 |
| N-H, oop bending | 698 | 707 | | 685 | 669 | 616 |
| O-H, stretch | 3449 | 3436 | | 3443 | 3415 | 3402 |
| b.Thermalanalysis: | | | | | | |
| Hydrated water | 0 | 10% | 42% | | 20% | 40% |
| Dehydration temperature, °C | | 109. | 139. | 110 | 110 | 122 |
| T$_m$, °C | 206 | 160 | 193,238 | 219 | . | 160. |
| T$_d$, °C | 209.8 | 340 | 341 | 223 | 335.7 | 331.8 |

γ-polyglutamic acid and its salts possess excellent water binding properties and moisture retention capacity, and their biochemical and biological functionalities are being explored for applications in cosmetic products and personal care products. γ-Polyglutamic acid and its salts were recently found to stimulate the growth of fibroblast cells, and show long-lasting moisture retention and good whitening results in skin care applications. The objectives in this invention are to provide an economical and very effective moisturizer formulation systems for use in cosmetic products and personal care products which possess excellent moisturizing capability, provide tender and smooth soft feeling, and improve the health of the skin.

The subject invention relates the use of a γ-polyglutamate hydrogel as a moisturizer in a cosmetic or personal care product, wherein said γ-polyglutamate hydrogel is prepared from γ-polyglutamate in Na+ form, γ-polyglutamate in K$^+$ form, γ-polyglutamate in NH$_4^+$ form, γ-polyglutamate in Mg$^{++}$ form, γ-polyglutamate in Ca$^{++}$ form, or a mixture of two or more thereof cross-linked with diglycerol polyglycidyl ether, polyglycerol polyglycidyl ether, sorbitol polyglycidyl ether, polyoxyethylene sorbitol polyglycidyl ether, polysorbitol polyglycidyl ether, or a mixture of two or more thereof, wherein the amount of said moisturizer is from 0.005 wt.% to 5 wt.% of the cosmetic or personal care product.

The subject invention further relates to a use as defined in the preceding paragraph, which is use of a γ-polyglutamate hydrogel and γ-polyglutamic acid (γ-PGA, H form), γ-polyglutamate in Na$^+$ form, γ-polyglutamate in K$^+$ form, γ-polyglutamate in NH$_4^+$ form, γ-polyglutamate in Mg$^{++}$ form, γ-polyglutamate in Ca$^{++}$ form, or a mixture of two or more thereof as a moisturizer in a cosmetic or personal care product, wherein said γ-polyglutamate hydrogel is prepared from γ-polyglutamate in Na$^+$ form, γ-polyglutamate in K$^+$ form, γ-polyglutamate in NH$_4^+$ form, γ-polyglutamate in Mg$^{++}$ form, γ-polyglutamate in Ca$^{++}$ form, or a mixture of two or more thereof cross-linked with diglycerol polyglycidyl ether, polyglycerol polyglycidyl ether, sorbitol polyglycidyl ether, polyoxyethylene sorbitol polyglycidyl ether, polysorbitol polyglycidyl ether, or a mixture of two or more thereof.

According to the subject invention, the γ-polyglutamic acid (γ-PGA, H form), γ-polyglutamate in Na$^+$ form, γ-polyglutamate in K$^+$ form, γ-polyglutamate in NH$_4^+$ form, γ-polyglutamate in Ca$^{++}$ form, γ-polyglutamate in Mg$^{++}$ form may independently have a low molecular weight ranging from 100,000 to 500,000 or a high molecular weight ranging from $10^6$ to $3 \times 10^6$. The cross-linked γ-polyglutamate hydrogel preferably has a molecular weight ranging from $15 \times 10^6$ to $200 \times 10^6$. The γ-polyglutamic acid (γ-PGA, H form), γ-polyglutamate in Na$^+$ form, γ-polyglutamate in K$^+$ form, γ-poly-glutamate in NH$_4^+$ form, γ-polyglutamate in Ca$^{++}$ form, and γ-polyglutamate in Mg$^{++}$ form utilized in the subject invention can be produced from submerged fermentation process using L-glutamic acid and glucose as main nutrients, or the extracts from natto the solid state fermented soybeans. Furthermore, the cross-linked γ-polyglutamate hydrogel can be produced from γ-polyglutamate in Na$^+$ form, γ-polyglutamate in K$^+$ form, γ-polyglutamate in NH$_4^+$ form, γ-polyglutamate in Ca$^{++}$ form, and γ-polyglutamate in Mg$^{++}$ form, or a mixture of two or more thereof cross-linked with poly-functional chemical cross-linking agents - diglycerol polyglycidyl ether, polyglycerol polyglycidyl ether, sorbitol polyglycidyl ether, polyoxyethylene sorbitol polyglycidyl ether, polysorbitol polyglycidyl ether, or a mixture of two or more thereof.

The amount of a moisturizer is from 0.005 wt.% to 5 wt.% of the cosmetic and personal care product, preferably from 0.08 wt.% to 1 wt.%. Moreover, the cosmetic and personal care product comprises, but not limited to, a hand-care, face-care, body-care, foot-care, head-care, and hair-care, nail-care, or mouth-care product.

**EXPERIMENTAL METHODS OF THE INVENTION**

Large quantity of γ-polyglutamic acid (γ-PGA, H form) and its salts (i.e., γ-polyglutamate in $Na^+$ form, γ-polyglutamate in $K^+$ form, γ-polyglutamate in $NH_4^+$ form, γ-polyglutamate in $Mg^{++}$ form, and γ-polyglutamate in $Ca^{++}$ form) can be produced in a submerged fermentation process with *Bacillus subtilis, Bacillus subtilis* var. natto (see JP 01-174397), or *Bacillus licheniformis* (see JP 11-343339) by using L-glutamic acid and glucose as main feed stocks. The microbial culture media contain carbon source, nitrogen source, inorganic minerals, and other nutrients in a proper quantity. Usually, L-glutamic acid is used at a concentration ranging from 3 to 12%, glucose at a concentration of 5-12%, citric acid at a concentration of 0.2 to 2% are used as partial carbon source; peptone and ammonium sulfate or urea are used as nitrogen sources; yeast extract is used as nutrient source; $Mn^{++}$, $Mg^{++}$ and NaCl are used as mineral sources. Under proper aeration and agitation, the culture temperature is maintained at 30 to 40 °C, and pH is maintained at 6-7.5 by using urea solution or sodium hydroxide solution; the culture time is normally a period of 48 to 84 hours. γ-Polyglutamic acid (γ-PGA) and its salts, (i.e., γ-polyglutamate in $Na^+$ form, γ-polyglutamate in $K^+$ form, γ-polyglutamate in $NH_4^+$ form, γ-polyglutamate in $Mg^{++}$ form, and γ-polyglutamate in $Ca^{++}$ form) are accumulated extracellularly.

γ-Polyglutamic acid (γ-PGA, H form) and its salts are normally extracted from the fermentation broth by a series of procedures including, ultra-centrifugation, or pressurized filtration to separate cells, then adding 3-4 times of ethanol to precipitate out γ-polyglutamic acid (γ-PGA, H form) and its salts,. The precipitates are redissolved in water, and another portion of ethanol is used to precipitate out γ-polyglutamic acid (γ-PGA, H form) and its salts. The dissolution-precipitaion steps are repeated several times in order to recover pure γ-polyglutamic acid (γ-PGA, H form) and its salts.

One approach to preparing a hydrogel which is not a hydrogel for use according to the invention, which is included here for reference purposes only, is as follows. γ-Polyglutamic acid (γ-PGA, H form) and its salts are normally dissolved in a proper solvent such as water, ethanol or methanol and pH is adjusted 5.0 to 7.5. The solution is then transferred to a proper radiation-permeable glass or plastic containers and evacuated, and irradiated with a gamma rays or electron beams at a total radiation dosage ranging from of 0.5 to 5.0.Mrad (see JP 11-343339, JP 2001-354542, and JP 06-322358), depending on the required quality of hydrogels. The commonly used gamma ray irradiation source is Cobalt 60 at an irradiation rate of 0.1 to 0.15 Mrad/Hr or an electron beam with a similar capacity. The hydrogels formed are then freeze-dried to produce dried cross-linked γ-polyglutamic acid (γ-PGA, H form) and its salts, which possess super water absorption capacity, are insoluble, and form colorless, transparent and biodegradable hydrogels when fully swell in water.

An approach to preparing a hydrogel which is for use according to the invention is as follows. γ-Polyglutamic acid (γ-PGA, H form) and its salts are normally dissolved in a proper solvent such as water, ethanol or methanol and pH is adjusted to 5.0 to 7.5. the properly selected multiple functional chemical cross-linking agents, namely diglycerol polyglycidyl ether, polyglycerol polyglycidyl ether, sorbitol polyglycidyl ether, polyoxyethylene sorbitol polyglycidyl ether, polysorbitol polyglycidyl ether, or a mixture of two or more thereof are added to the solution under constantly stirring, at a dose rate ranging from 0.1 to 20% of the weight of γ-polyglutamic acid (γ-PGA, H form) and its salts, depending on the type of cross-linking agents and the quality of hydrogels required. The gelling reaction is normally completed within 1 to 4 hours at a reaction temperature from 50 to 120 °C depending on the equipment and conditions used. The hydrogels formed are then freeze-dried to produce dried cross-linked γ-polyglutamic acid (γ-PGA, H form) and its salts, γ-polyglutamate hydrogels, which possess super water absorption capacity, are insoluble, and form colorless, transparent and biodegradable hydrogels when fully swell in water.

The cross-linked hydrogels for use according to the invention thus produced with poly-functional chemical cross-linking agents possess super water absorption and water retention capacity, form a tender, smooth, gentle soft film on skin or hair, and are especially suitable for use in cosmetic and personal care products for skin care and hair care, including hand-care, face-care, body-care, foot-care, head-care, and hair-care, nail-care, mouth-care. The amounts of the cross-linked hydrogels in cosmetic and personal care products range from 0.005 to 5 wt.% in the final products, depending on the product quality required.

Small and middle molecular weight γ-polyglutamic acid (γ-PGA) and its salts, with molecular weight ranging from 5000 to 900,000 can be produced by controlled acidic-hydrolysis at a specific selected reaction conditions of pH, temperature, reaction time and concentration of γ-polyglutamic acid (γ-PGA, H form). The pH can be from pH 1.5 to 5.5 with proper acidulant such as HCl, $H_2SO_4$, or other organic acids, the hydrolysis temperature can be controlled in the range from 50 to 110°C, the reaction time from 0.5 to 5 hours, and the concentration of γ-polyglutamic acid (γ-PGA, H form) with molecular weight of $1 \times 10^6$ or higher can be any concentration (as required) (see JP 06-322358, Characteristic Properties of N-Carboxybutyl Chitosan. Carbohydr. Polym. 11:307-320, 1989, Muzzarelli, R. A. A. et al., and Appraisel of the Safety of Chemicals in Food, Drugs and Cosmetics. Pharmacolo. 93:377-392,1948, Draize, F. et al.). After the reaction is completed, further purification with dialysis or membrane filtration and drying are necessary to produce high purity small and middle molecular weight γ-polyglutamic acid (γ-PGA, H form) and its salts, of choice. The acid-hydrolysis rate is faster at lower pH, higher temperature, and higher concentration of γ-polyglutamic acid (γ-PGA). The γ-polyglutamate salts can be produced by reaction of selected γ-polyglutamic acid (γ-PGA) with basic hydroxide solution or oxide of the metal ions of $Na^+$, $K^+$, $NH_4^+$, $Ca^{++}$ or $Mg^{++}$ of choice, and pH adjusted to desired condition from 5.0 to 7.2 as required.

**EXPERIMENTAL EXAMPLE**

In order to further explain this invention in detail, experimental examples are presented in the following to show how this invention can be utilized to achieve the best moisturizing effect and adding health values to the cosmetic and personal care products. But the scope of this invention is not limited by these experimental examples.

**EXPERIMENTAL EXAMPLE 1**

In this experimental example, the standard skin vital moisture creams with the following formulation were prepared to demonstrate the effectiveness of water retention by γ-polyglutamate in $Mg^{++}$ form (Reference Examples) and γ-polyglutamate hydrogel for use according to the invention (prepared from γ-polyglutamate in $Na^+$ form). Propylene glycol was used as control for comparison, hyaluronic acid (HA) was used as relative reference, the small molecular weight γ-polyglutamate in $Mg^{++}$ form with 200,000 to 400,000 daltons (designated as LM), high molecular weight γ-polyglutamate in $Mg^{++}$ form with $1.15 \times 10^6$ to $1.35 \times 10^6$ daltons (designated as HM), and γ-polyglutamate hydrogel for use according to the invention (prepared from γ-polyglutamate in $Na^+$ form) with molecular weight from $15 \times 10^6$ to $100 \times 10^6$ or higher (cross-linked with polyglycerol polyglicidyl ether) were used as super moisturizers. The species and proprotions of the ingredient contained in the exemplified cream formulations are listed in Table 2.

Table 2: Skin vital moisture cream formulation.

| INGREDIENT | PERCENTAGE,% | | | | |
|---|---|---|---|---|---|
| | CONTROL | A | B | C | D |
| Stearic Acid | 5.0 | 5.0 | 5.0 | 5.0 | 5.0 |
| Stearyl Alcohol | 4.0 | 4.0 | 4.0 | 4.0 | 4.0 |
| Wichenol 158 | 6.0 | 6.0 | 6.0 | 6.0 | 6.0 |
| KOH | 0.2 | 0.2 | 0.2 | 0.2 | 0.2 |
| Propylene Glycol | 5.0 | --- | --- | --- | --- |
| γ-Polyglutamate in $Mg^{++}$ form HM | --- | 0.1 | --- | --- | --- |
| γ-Polyglutamate in $Mg^{++}$ form LM | --- | --- | 0.1 | --- | --- |
| γ-Polyglutamate Hydrogel (prepared from γ-polyglutamate in $Na^+$ form) 4%1CL | --- | --- | --- | 0.1 | --- |
| Hyaluronic acid (HA) | --- | --- | --- | --- | 0.1 |
| GMS 1330 | 2.0 | 2.0 | 2.0 | 2.0 | 2.0 |
| Methylparaben | 0.4 | 0.4 | 0.4 | 0.4 | 0.4 |
| De-ionized Water | 77.2 | 82.1 | 82.1 | 82.1 | 82.1 |
| Vitamin E | 0.2 | 0.2 | 0.2 | 0.2 | 0.2 |
| Note: The hydrogel 4%1CL is the sample code of hydrogel used in the experiment. | | | | | |

The hydrogel was made with 4% γ-polyglutamate in $Na^+$ form and 1% polyglycerol polyglycidyl ether

**EXPERIMENTAL EXAMPLE 2**

The cosmetic products from experimental example 1 were evaluated for the effectiveness of water retention on skin. A sample of 0.2g from each of the 5 products was evenly spread over an area of about 25 $cm^2$ on the inside

skin surface of a test panelist's arm. A group of 10 panelists participated in this test. The water retention on the skin surface was measured at 23 °C under an environment of relative humidity of 60%, with a probe of Skin Analysis SHP88 made by Courage $^+$Khazaka Electronic Gmbh, Germany. The effectiveness of water retention was expressed in terms of capacitance increase ratio (%), as shown in Table 3. The results show a much better long lasting water retention quality for products containing γ-polyglutamate in Mg$^{++}$ form and γ-polyglutamate hydrogel for use according to the invention (prepared from γ-polyglutamate in Na$^+$ form).

Table 3. The change of moisture retention over time after applying vital moisture creams, was expressed in terms of capacitance increase ratio, %, measured at 22°C and RH 65%.

| Moisturizer used | Capacitance Increase Ratio, % | | | | | | |
|---|---|---|---|---|---|---|---|
| | Time, min. | | | | | | |
| | 0 | 5 | 10 | 20 | 40 | 80 | 120 |
| 5% Propylene Glycol as control | 0 | 105 | 57 | 51 | 40 | 38 | 36 |
| 0.1% Hyaluronic acid (HA)* | 0 | 121 | 75 | 53 | 42 | 41 | 40 |
| 0.1% γ-Polyglutamate in Mg$^{++}$ form HM* | 0 | 180 | 118 | 79 | 54 | 48 | 45 |
| 0.1 % γ-Polyglutamate in Mg$^{++}$ form LM* | 0 | 138 | 98 | 70 | 46 | 45 | 43 |
| 0.1% γ-Polyglutamate Hydrogel (prepared from γ-polyglutamate in Na$^+$ form) 4%1CL | 0 | 128 | 78 | 56 | 44 | 43 | 42 |
| *Reference Examples | | | | | | | |

## EXPERIMENTAL EXAMPLE 3

The cosmetic products from experimental example 1 were also evaluated for the effectiveness of improving the elasticity of skin. A sample of 0.25g from each of the 5 products was evenly spread over an area of 25 cm$^2$ on the outside skin surface of a test panelist's arm, once a day and continuously for a period of 1 month. 10 panelists participated in the test. The apparent skin elasticity was measured at 23 °C and under relative humidity (RH) of 60%, with a probe of Cutometer SEM 575 (Courage$^+$ Khaazaka Electronic Gmbh, German), once per week and expressed in terms of the apparent elasticity index R2 value. The higher the apparent elasticity index R2 value, the better the skin elasticity. The results are shown in Table 4. The results show that the Reference Example cosmetic formulations containing γ-polyglutamate in Mg$^{++}$ form (HM) and γ-polyglutamate in Mg$^{++}$ form (LM), and the cosmetic formulation for use according to the invention containing γ-polyglutamate hydrogel (prepared from γ-polyglutamate in Na$^+$ form) 4%1 CL are much better than those containing hyaluronic acid (HA) or propylene glycol in improving skin elasticity. The γ-polyglutamate hydrogel for use according to the invention (prepared from γ-polyglutamate in Na$^+$ form) shows the best results in improving the skin elasticity.

Table 4. The changes in skin elasticity over time after applying vital moisture cream. The apparent elasticity index was expressed in R2 value, and the relative apparent elasticity was expressed in R2/(R2)$_0$, %.

| Moisturizer used | Relative Apparent Elasticity, R2/(R2)$_0$,% Time, week | | | | | |
|---|---|---|---|---|---|---|
| | (R2)$_0$ | 0 | 1 | 2 | 3 | 4 |
| 5% Propylene glycol (control) | 0.760 | 100 | 103.3 | 104.5 | 104.7 | 104.6 |
| 0.1% Hyaluronic acid (HA)* | 0.800 | 100 | 103.5 | 105.6 | 105.5 | 105.5 |
| 0.1% γ-Polyglutamate in Mg$^{++}$ form HM* | 0.825 | 100 | 105.7 | 106.1 | 106.7 | 106.7 |
| 0.1% γ-Polyglutamate in Mg$^{++}$ form LM* | 0.850 | 100 | 103.3 | 105.4 | 105.5 | 105.6 |
| 0.1% γ-Polyglutamate Hydrogel (prepared from γ-polyglutamate in Na$^+$ form) 4%1CL | 0.780 | 100 | 103.5 | 107.7 | 110.5 | 110.3 |
| *Reference Examples | | | | | | |

**EXPERIMENTAL EXAMPLE 4 (Reference Example)**

In this experimental example, a standard facial moisture mask formulation with the following formulations were prepared to demonstrate the effectiveness of water retention by $\gamma$-polyglutamate in $Ca^{++}$ form and $\gamma$-polyglutamate hydrogel (prepared from $\gamma$-polyglutamate in $Na^+$ form). The small molecular weight $\gamma$-polyglutamate in $Ca^+$ form with 200,000 to 400,000 daltons (designated as LM), high molecular weight $\gamma$-polyglutamate in $Ca^{++}$ form with $1.15 \times 10^6$ to $1.35 \times 10^6$ daltons (designated as HM), and $\gamma$-polyglutamate hydrogel (prepared from $\gamma$-polyglutamate in $Na^+$ form) with molecular weight from $15 \times 10^6$ to $100 \times 10^6$ or higher (cross-linked with gamma ray irradiation) were used as super moisturizers. The species and proportions of the ingredients contained in the exemplified facial moisture mask formulation are listed in Table 5.

Table 5. Facial Moisture Mask Formulation

| INGREDIENT | PERCENTAGE, % | | | | |
|---|---|---|---|---|---|
| | CONTROL | A | B | C | D |
| Polyethylene Glycol | 15.0 | 15.0 | 15.0 | 15.0 | 15.0 |
| Methylcellulose | 2.0 | 2.0 | 2.0 | 2.0 | 2.0 |
| 1,3-Butyl Glycol | 5.0 | --- | ---- | --- | --- |
| Ethanol | 12.0 | 12.0 | 12.0 | 12.0 | 12.0 |
| Methylparaben | 0.4 | 0.4 | 0.4 | 0.4 | 0.4 |
| Sodium Citrate | q.s. | q.s. | q.s. | q.s. | q.s. |
| $\gamma$-Polyglutamate in $Ca^{++}$ form HM | ---- | 0.15 | | | |
| $\gamma$-Polyglutamate in $Ca^{++}$ from LM | ---- | | 0.15 | | |
| $\gamma$-Polyglutamate Hydrogel (prepared from $\gamma$-polyglutamate in $Na^+$ form) 2%2M | ---- | | | 0.15 | |
| $\gamma$-Polyglutamate Hydrogel (prepared from $\gamma$-polyglutamate in $Na^+$ from) 6%1M | ---- | | | | 0.15 |
| POE Oleyl Alcohol Ether | 0.5 | 0.5 | 0.5 | 0.5 | 0.5 |
| De-ionized Water | 65.1 | 69.9 | 69.9 | 69.9 | 69.9 |
| Note: The Hydrogel 2%2M and Hydrogel 6%1M are the sample codes of hydrogel used in the experiment. Hydrogel 2%2M represents the hydrogel was made of 2% $\gamma$-polyglutamate in $Na^+$ form and irradiated with $\gamma$-ray at a dosage of 2 Mrad, and Hydrogel 6%1M was made of 6% $\gamma$-polyglutamate in $Na^+$ form and irradiated with $\gamma$-ray at a dosage of 1 Mrad. | | | | | |

**EXPERIMENTAL EXAMPLE 5 (Reference Example)**

The samples of 0.2 grams from experimental example 4 were taken and evenly spread over an area of 25 $cm^2$ on the outside skin surface of the arm of a test panelist, and the film formation time was closely observed and recorded (see Characteristic Properties of N-Carboxybutyl Chitosan. Carbohydr. Polym. 11:307-320, 1989, Muzzarelli, R. A. A. et al.) at 23 °C under an environment of relative humidity (RH) of 65%. A group of 10 panelists participated in this test. In a separate experiment, the mask film formed after 15 minutes was peeled off, then the pH value of the skin was measured at 23°C under an environment of relative humidity of 65% with a probe of the Skin Analysis SHP88 of Courage$^+$Khazaka Electronic Gmbh, Germany. The moisture retention of the skin was immediately measured at 23°C under an environment of relative humidity of 65% with a probe of the Skin Analysis SHP88, and expressed in unit of capacitance increase ratio,%.

The moisture mask samples of 25 $cm^2$ were taken from experimental example 4 and patched evenly on the outside surface of the upper arm skin of a test panelist. A group of 10 panelists participated in this test. After 24 hours, then the mask samples were removed and the skin was observed carefully for any degree of irritation, and the results were recorded according to Draize method (see Appraisel of the Safety of Chemicals in Food, Drugs and Cosmetics. Pharmacolo. 93:377-392, 1948, Draize, F. et al.). The results are shown in Table 6.

Table 6. The film formation time, capacity increase ratio (%), the pH value of the skin, and the Draize score after applying facial moisture masks

| | 1,3-BG Controll | γ-polyglutamate in Ca$^{++}$ form HM | γ-polyglutamate in Ca$^{++}$ form LM | Hydrogel 2%2M | Hydrogel 6%1M |
|---|---|---|---|---|---|
| Film Formation Time, min. | 13.2 | 14.2 | 13.5 | 12.0 | 14.6 |
| Capacitance Increase Ratio,% | 73.2 | 89.6 | 83.0 | 94.7 | 83.9 |
| pH value, before using mask | 5.8 | 6.1 | 6.0 | 6.0 | 6.0 |
| pH value, after using mask | 6.0 | 6.3 | 6.5 | 6.4 | 6.5 |
| Draize Score | 0 | 0 | 0 | 0 | 0 |
| Note: Hydrogel 2%2M and Hydrogel 6%1M are the sample codes of hydrogels used in this experiment. | | | | | |

Table 6 shows that the skin water retention as expressed by the capacitance increase ratio is much better with facial moisture mask containing γ-polyglutamate in Ca$^{++}$ form(HM), 89.6%, γ-polyglutamate in Ca$^{++}$ form (LM), 83.0%, and γ-polyglutamate hydrogel (prepared from γ-polyglutamate in Na$^{+}$ from) (2%2M), 94.7%, and γ-polyglutamate hydrogel (prepared from γ-polyglutamate in Na$^{+}$ from) (6%1M), 83.9% than with the control, 73.2%. While the skin pH value is maintained within the weak acid range, below pH 6.5, as compared to pH 6.0 for the control, the Draize score of "zero" suggests that γ-polyglutamate in Ca$^{++}$ form and γ-polyglutamate hydrogels (prepared from γ-polyglutamate in Na$^{+}$ form) do not cause any rash or irritation to the skin.

## EXPERIMENTAL EXAMPLE 6 (Reference Example)

In this experimental example, the standard skin vital moisture creams with the following formulations were prepared to demonstrate the effectiveness of water retention by γ-polyglutamate in K$^{+}$ form and γ-polyglutamate hydrogel (prepared from γ-polyglutamate in K$^{+}$ form). Glycerol was used as control for comparison, high molecular weight γ-polyglutamate in K$^{+}$ form with $1.15 \times 10^{6}$ to $1.45 \times 10^{6}$ daltons (designated as HM), and γ-polyglutamate hydrogels (prepared from γ-polyglutamate in K$^{+}$ form) with molecular weight from $15 \times 10^{6}$ to $100 \times 10^{6}$ or higher (cross-linked with gamma ray irradiation) were used as super moisturizers. The species and proportions of the ingredients contained in the exemplified cream formulation are listed in **Table 7.**

Table 7: Skin moisture cream formulation.

| INGREDIENT | PERCENTAGE,% | | | |
|---|---|---|---|---|
| | CONTROL | A | B | C |
| Glyceryl Stearate | 4.0 | 4.0 | 4.0 | 4.0 |
| Stearyl Alcohol | 2.0 | 2.0 | 2.0 | 2.0 |
| Ethylhexyl Stearate | 8.5 | 8.5 | 8.5 | 8.5 |
| Capric/Caprylic Triglyceride | 8.5 | 8.5 | 8.5 | 8.5 |
| Ceteareth-25 | 2.0 | 2.5 | 2.5 | 2.5 |
| Glycerol | 5.0 | --- | --- | --- |
| γ-Polyglutamate in K$^{+}$ from HM | --- | 0.2 | --- | --- |
| γ-Polyglutamate Hydrogel (prepared from γ-polyglutamate in K$^{+}$ form) 6%1M | --- | --- | 0.2 | --- |
| Natural Betaine | --- | --- | --- | 2.0 |
| Na-Benzoate, Methylparaben | 0.4 | 0.4 | 0.4 | 0.4 |
| De-ionized Water | 69.5 | 74.3 | 74.3 | 72.5 |

(continued)

| INGREDIENT | PERCENTAGE,% | | | |
| | CONTROL | A | B | C |
| --- | --- | --- | --- | --- |
| Vitamin E | 0.1 | 0.1 | 0.1 | 0.1 |

Note: .The Hydrogel, 6%1M is the sample code of hydrogel used in the experiment.

## EXPERIMENTAL EXAMPLE 7 (Reference Example)

The cosmetic products from experimental example 6 were evaluated for the effectiveness of skin moisturization. A group of 5 test panelists participated in this experiment. The panelists were first put in a room with a temperature at 22 °C and relative humidity (RH) 60% for 15 minutes. A probe of the corneometer HM99 of Courage⁺Khazaka Electronic Gmbh, Germany was used to measure the skin moisture of the outside skin surface of the panelist's arm to determine the base line as (CU1). A sample of 0.2g from the formulation of experimental example 6 was evenly spread over an area of 25 cm$^2$ of the skin surface for 2 minutes before the excess formulation sample was removed. After 2 hours the skin moisture was measured again and recorded as (CU2). Before the measurement the panelists were placed in a room with temperature at 22 °C and RH 60% for 15 minutes. The atteration of skin moisture is then calculated as ΔCU=CU2-CU1. The results of increase in skin moisture are shown in Table 8.

Table 8. Relative Increase in Skin Moisture

| Moisturizer used | Increase in Skin Moisture ΔCU | Relative ΔCU % | New Film Property |
| --- | --- | --- | --- |
| 5% Glycerol | 7.5 | 100 | No change |
| 0.2% γ-Polyglutamate in K⁺ form HM | 11.0 | 147 | Smooth feel |
| 0.2% γ-Polyglutamate Hydrogel (prepared from γ-polyglutamate in K⁺ form) 6% 1M | 13.5 | 180 | Soft, tender, slippery feel |
| 2% Natural Betaine | 12.3 | 164 | No change |

Note: .The Hydrogel 6%1M is the sample code of hydrogel, which was made of 6% γ-polyglutamate in K⁺ form and irradiated with γ-ray at a dosage of 1 Mrad.

Table 8 shows that γ-polyglutamate in K⁺ form and γ-polyglutamate hydrogel (prepared from γ-polyglutamate in K⁺ form) provide better skin moisture increase than the conventional moisturizers even at a use level of more than 10 time less, with the Relative Increase in Skin Moisture, ΔCU: 147% for 0.2% γ-polyglutamate in K⁺ form, 180% for 0.2% γ-polyglutamate hydrogel (prepared from γ-polyglutamate in K⁺ form), as compared to 100% for 5% Glycerol, and 164% for 2% Natural Betaine. γ-Polyglutamate in K⁺ form and γ-polyglutamate hydrogel (prepared from γ-polyglutamate in K⁺ form) also form a new matrix film on the outside of skin surface to provide a comfortable, soft, tender, and smooth quality, which are desirable to most users.

## EXPERIMENTAL EXAMPLE 8

In this experiment, the standard skin vital moisture creams with the following formulations were prepared to demonstrate the effectiveness of water retention by γ-polyglutamate in Mg⁺⁺ form (Reference Example) and γ-polyglutamate hydrogels for use according to the invention (prepared from γ-polyglutamate in Mg⁺⁺ form). Glycerol was used as control for comparison, high molecular weight γ-polyglutamate in Mg⁺⁺ form with $1.15 \times 10^6$ to $1.45 \times 10^6$ daltons (designated as HM), and γ-polyglutamate hydrogel for use according to the invention (prepared from γ-polyglutamate in Mg⁺⁺ form) with molecular weight from $15 \times 10^6$ to $100 \times 10^6$ or higher (cross-linked with glycerol-based cross-linking agent) were used as super moisturizers. The species and proportions of the ingredients contained in the exemplified cream formulation are listed in Table 9.

Table 9. Skin moisture cream formulation.

| Ingredient | Control,% | A,% | B,% |
| --- | --- | --- | --- |
| Glyceryl Stearate | 4.0 | 4.0 | 4.0 |

(continued)

| Ingredient | Control,% | A,% | B,% |
|---|---|---|---|
| Stearyl Alcohol | 2.0 | 2.0 | 2.0 |
| Ethylhexyl Stearate | 8.5 | 8.5 | 8.5 |
| Capric/Caprylic Triglyceride | 8.5 | 8.5 | 8.5 |
| Ceteareth-25 | 2.0 | 2.0 | 2.0 |
| Glycerol | 6.5 | --- | --- |
| $\gamma$-Polyglutamate in Mg$^{++}$ form HM | --- | 0.5 | --- |
| $\gamma$-Polyglutamate Hydrogel (prepared from $\gamma$-polyglutamate in Mg$^{++}$ from) 6%1M | --- | --- | 0.5 |
| Na-Benzoate, Methylparaben | 0.4 | 0.4 | 0.4 |
| De-ionized Water | 68.0 | 74.0 | 74.0 |
| Vitamin E | 0.1 | 0.1 | 0.1 |
| Note: .The Hydrogel 6%1M is the sample code of hydrogel, which was made of 6%$\gamma$-Polyglutamate in Mg$^{++}$ form and irradiated with $\gamma$-ray at a dosage of 1 Mrad. | | | |

## EXPERIMENTAL EXAMPLE 9

The cosmetic products from experimental example 8 were evaluated for the effectiveness of reducing water evaporation loss and retention of moisture in skin. Pig skin was used for this experiment. The skin moisture was measured at 23 °C under an environment of relative humidity (RH) of 60% with a probe of corneometer HM99 of Courage$^+$Khazaka Electronic Gmbh, Germany. A similar procedure in experimental example 7 followed. The results of Trans-Epidermal-Water-Loss (TEWL) and skin moisture retention are shown in Tables 10a and 10b.

Table 10a. Test results with *ex-vivo* pig skin: skin moisture retention by corneometry.

| With moisturizer used | Difference Corneometry, % Time, hour | | | | |
|---|---|---|---|---|---|
| | -1 | 0 | 1 | 2 | 4 |
| 6.5% Glycerol | 100 | 48.. | 32 | 20 | 17 |
| 0.5% $\gamma$-Polyglutamate in Mg$^{++}$ form HM* | 100 | 67 | 36 | 22 | 19 |
| 0.5% $\gamma$-Polyglutamate Hydrogel (prepared from $\gamma$-polyglutamate in Mg$^{++}$ form) 6%1M | 100 | 84 | 55 | 34 | 26 |
| *Reference Example | | | | | |

The skin moisture retention under the same climate conditions (23°C and RH 60%) showed a leveling off about in 4 hours after application of the formulation. The cosmetic formulation for use according to the invention containing 0.5% $\gamma$-polyglutamate hydrogel (prepared from $\gamma$-polyglutamate in Mg$^{++}$ form) shows best skin moisture retention of 84% at hour 1 and 34% at hour 2, as compared to 36% at hour 1 and 22% at hour 2 for that containing 0.5 $\gamma$-polyglutamate in Mg$^{++}$ form, and only 32% at hour 1 and 20% at hour 2 for that containing 6.5% glycerol.

Table 10b. Test results with *ex-vivo* pig skin: Trans-Epidermal-Water-Loss(TEWL)

| With moisturizer used | Trans-Epidermal-Water-Loss (TEWL) | | | | |
|---|---|---|---|---|---|
| | | | | Time, hour | |
| | -1 | 0 | 1 | 2 | 4 |
| 6.5% Glycerol | 24.7 | 21.0. | 19.5 | 19.0 | 17.5 |
| 0.5% $\gamma$-Polyglutamate in Mg$^{++}$ form, HM* | 24.0 | 19.5 | 18.5 | 17.5 | 17.0 |

(continued)

| With moisturizer used | Trans-Epidermal-Water-Loss (TEWL) | | | | |
|---|---|---|---|---|---|
| | Time, hour | | | | |
| | -1 | 0 | 1 | 2 | 4 |
| 0.5% γ-Polyglutamate Hydrogel (prepared from γ-polyglutamate in Mg$^{++}$ from), 6%1M | 19.0 | 15.0 | 13.0 | 12.5 | 12.5 |
| *Reference Example | | | | | |

Obviously, the less TEWL the less water loss by evaporation of water through skin. The TEWL also shows a leveling off at 4 hours after application of the moisture formulation. The formulation containing 0.5% γ-polyglutamate hydrogel for use according to the invention (prepared from γ-polyglutamate in Mg$^{++}$ form) has the lowest TEWL value of 13.0 at hour 1 and 12.5 at hour 2, as compared to 19.5 at hour 1 and 17.5 at hour 2 for that containing 0.5% γ-polyglutamate in Mg$^{++}$ form, and 19.5 at hour 1 and 10.0 at hour 2 for that containing 6.5% glycerol. Apparently, γ-polyglutamate in Mg$^{++}$ form and γ-polyglutamate hydrogel (prepared from γ-polyglutamate in Mg$^{++}$ form) enhance the skin moisturization and reduce the TEWL, which means a better moisturizer and a better water barrier for the cosmetic and personal care products.

## EXPERIMENTAL EXAMPLE 10 (Reference Example)

In this set of *in vitro* cell culture experiments, a monolayer of cell line L-929 fibroblasts was grown to near confluency of about $2 \times 10^5$ cells in DMEM (Dulbecco's Modified Eagle Medium) with 10% fetal bovine serum, in each well of a 24 -well cell culture plate. The control was the monolayer cultured in 1 ml of DMEM. The sample containing 10mg of small molecular weight γ-polyglutamatein Na$^+$ form with 200,000 to 350,000 daltons (designated as LM) in 1 ml of DMEM was used as test extract. Triplicate cultures were prepared. The cultures were incubated for 24 hours at 37°C with 5% $CO_2$. Then the monolayer was examined microscopically, trypsinized and the cell density was counted. The respective cell number within the monolayer are listed in Table 11.

Table 11. Cell Layer Density.

| Sample | Total number of cells within monolayer (x10$^4$) | | | |
|---|---|---|---|---|
| | #1 | #2 | #3 | Mean (n=3) |
| Control | 3.9 ± 0.3 | 6.7 ± 0.3 | 4.6 ± 0.3 | 5.1 ± 1.5 |
| Test Extract, with 1% γ-polyglutamate in Na$^+$ form, LM | 9.6 ± 0.4 | 7.7 ± 0.2 | 7.6 ± 0.6 | 8.3 ± 1.1 |

The results in Table 11 shows that the small molecular weight γ-polyglutamate in Na$^+$ form,LM, with 200,000 to 350,000 daltons (designated as LM) stimulated the growth of fibroblast cell, indicating that γ-polyglutamate in Na$^+$ form, LM helps the growth of fibroblast cells.

## EXPERIMENTAL EXAMPLE 11 (Reference Example)

Although the formation of black pigment melanin on skin is complicated, it is generally realized that tyrosine is oxidized to DOPA (Dihydroxy Phenyalanine) by the catalysis of tyrosinase from melansome of melanocyte. The DOPA is further oxidized and finally converted into colored melanin-protein complex. The small molecular weight γ-polyglutamate in Na$^+$ form with 200,000 to 350,000 daltons (designated as LM) and high molecular weight γ-polyglutamate in Na$^+$ form with $1.15 \times 10^6$ to $1.35 \times 10^6$ daltons (designated as HM) were used in this experiment to determine the effectiveness in inhibiting the tyrosinase activity.

a. Determination of total absorbency at wavelength of 475 nm:

To a 25 ml sample test tube, add 0.9 ml of 0.1 M phosphate buffer solution (pH 6.8), 1 ml of γ-polyglutamate (Na$^+$ form) sample and 1ml of 0.25 mg/ml tyrosine solution and mix well, then incubate at 37°C for 10 minutes. Add 0.1 ml of 8.55 unit/ml tyrosinase solution, mix well and re-incubate the mixture at 37°C for another 25 minutes. Sample of the reaction solution is then taken for measuring the absorbency at 475 nm, and recorded as At.

b. Determination of the blank absorbance of tyrosinase solutuion:

Replace 0.1 ml of buffer solution with tyrosinase solution, and repeat the same procedure in item (a), and record the absorbency at 475 nm as A1.

c. Replace 1 ml sample solution with buffer solution, and repeat the same procedure in item (a), and record the absorbance at 475 nm as Ab.

d. Determination of total blank absorbance of test solution:

Replace 1 ml sample solution with buffer solution, and 0.1 ml of tyrosinase solution with buffer solution, and repeat the procedure in item (a), and record the absorbency at 475 nm as Ao.

e. Calculation of the inhibition of tyrosinase activity:

$$\text{Inhibition of tyrosinase activity (\%)} = \frac{(Ab-Ao) - (At-A1)}{(Ab - Ao)} \times 100\%$$

The results on inhibition of tyrosinase activity (%) by γ-polyglutamate (Na+ form) are summarized in Table 12.

Table 12. Whitening effectiveness of γ-polyglutamate in Na+ form on inhibiting tyrosinase activity.

| Sample | Inhibition of Tyrosinase Activity, % |
|---|---|
| 0.5% γ-polyglutamate in Na+ form HM | 52.6 |
| 1.5% γ-polyglutamate in Na+ form HM | 65.1 |
| 1.0% γ-polyglutamate in Na+ form LM | 34.3 |
| 1.5% γ-polyglutamate in Na+ form LM | 48.6 |
| 1.0% Kojic Acid | 100.0 |
| 1.0% Vitamin C | 99.4 |

Table 12 shows that both γ-polyglutamate in Na+ form HM and γ-polyglutamate in Na+ form LM are relatively effective whitening agents for inhibiting the tyrosinase activity, with 0.5% γ-polyglutamate in Na+ form HM inhibiting 52.6% and 1.0% γ-polyglutamate in Na+ form LM inhibiting 34.3%, as compared to 100.0% for 1.0% Kojic Acid and 99.4% for 1.0% Vitamin C. The larger molecular weight γ-polyglutamate in Na+ form HM has better whitening effect than the smaller molecular weight γ-polyglutamate in Na+ form LM.

**Claims**

1. Use of a γ-polyglutamate hydrogel as a moisturizer in a cosmetic or personal care product, wherein said γ-polyglutamate hydrogel is prepared from γ- polyglutamate in Na+ form, γ-polyglutamate in K+ form, γ-polyglutamate in NH4+ form, γ-polyglutamate in Mg++ form, γ-polyglutamate in Ca++ form, or a mixture of two or more thereof cross-linked with diglycerol polyglycidyl ether, polyglycerol polyglycidyl ether, sorbitol polyglycidyl ether, polyoxyethylene sorbitol polyglycidyl ether, polysorbitol polyglycidyl ether, or a mixture of two or more thereof, wherein the amount of said moisturizer is from 0.005 wt.% to 5 wt.% of the cosmetic or personal care product.

2. Use according to claim 1 of a γ-polyglutamate hydrogel and γ-polyglutamic acid (γ-PGA, H form), γ-polyglutamate in Na+ form, γ-polyglutamate in K+ form, γ-polyglutamate in NH4+ form, γ-polyglutamate in Mg++ form, γ-polyglutamate in Ca++ form, or a mixture of two or more thereof as a moisturizer in a cosmetic or personal care product.

3. Use of any one of the preceding claims, wherein said γ-polyglutamate hydrogel has a molecular weight of from 15x10^6 to 200x10^6.

4. Use of any one of the preceding claims, wherein said γ-polyglutamic acid (γ-PGA, H form), γ-polyglutamate in Na+

form, γ-polyglutamate in K$^+$ form, γ-polyglutamate in NH$_4$$^+$ form, γ-polyglutamate in Mg$^{++}$ form, and γ-polyglutamate in Ca$^{++}$ form independently have a low molecular weight of from 100,000 to 500,000 or a high-molecular weight of from 10$^6$ to 3x10$^6$.

5. Use of any one of the preceding claims, wherein the product is a hand-care, face-care, body-care, foot-care, head-care, hair-care, nail-care, or mouth-care product.

6. Use according to claim 1, wherein the amount of said moisturizer is from 0.08 wt.% to 1 wt.% of said product.

**Patentansprüche**

1. Verwendung eines γ-Polyglutamat-Hydrogels als ein Feuchtigkeitsspender in einem kosmetischen Produkt oder Körperpflegemittel, wobei das γ-Polyglutamat-Hydrogel hergestellt ist aus γ-Polyglutamat in Na$^+$-Form, γ-Polyglutamat in K$^+$-Form, γ-Polyglutamat in NH$_4$$^+$-Form, γ-Polyglutamat in Mg$^{++}$-Form, γ-Polyglutamat in Ca$^{++}$-Form oder einem Gemisch von zweien oder mehreren davon, vernetzt mit Diglycerolpolyglycidylether, Polyglycerolpolyglycidylether, Sorbitolpolyglycidylether, Polyoxyethylensorbitolpolyglycidylether, Polysorbitolpolyglycidylether oder einem Gemisch von zweien oder mehreren davon, wobei die Menge des Feuchtigkeitsspenders im Bereich von 0,005 Gew.-% bis 5 Gew.-% des kosmetischen Produktes oder Körperpflegemittels beträgt.

2. Verwendung nach Anspruch 1 eines γ-Polyglutamat-Hydrogels und γ-Polyglutaminsäure (γ-PGA, H-Form), γ-Polyglutamat in Na$^+$-Form, γ-Polyglutamat in K$^+$-Form, γ-Polyglutamat in NH$_4$$^+$-Form, γ-Polyglutamat in Mg$^{++}$-Form, γ-Polyglutamat in Ca$^{++}$-Form oder ein Gemisch von zweien oder mehreren davon, als ein Feuchtigkeitsspender in einem kosmetischen Produkt oder Körperpflegemittel.

3. Verwendung nach einem der vorstehenden Ansprüche, wobei das γ-Polyglutamat-Hydrogel ein Molekulargewicht von 15x10$^6$ bis 200x10$^6$ aufweist.

4. Verwendung nach einem der vorstehenden Ansprüche, wobei die γ-Polyglutaminsäure (γ-PGA, H-Form), γ-Polyglutamat in Na$^+$-Form, γ-Polyglutamat in K$^+$-Form, γ-Polyglutamat in NH$_4$$^+$-Form, γ-Polyglutamat in Mg$^{++}$-Form und γ-Polyglutamat in Ca$^{++}$-Form unabhängig ein niedriges Molekulargewicht von 100.000 bis 500.000 oder ein hohes Molekulargewicht von 10$^6$ bis 3x10$^6$ aufweisen.

5. Verwendung nach einem der vorstehenden Ansprüche, wobei das Produkt ein Handpflege-, Gesichtspflege-, Körperpflege-, Fußpflege-, Kopfpflege-, Haarpflege-, Nagelpflege- oder Mundpflegemittel ist.

6. Verwendung nach Anspruch 1, wobei die Menge des Feuchtigkeitsspenders von 0,08 Gew.-% bis 1 Gew.-% des Produkts beträgt.

**Revendications**

1. Utilisation d'un hydrogel de poly-γ-glutamate comme agent hydratant dans un produit cosmétique ou un produit de soin personnel, lequel hydrogel de poly-γ-glutamate est préparé à partir de poly-γ-glutamate sous forme Na$^+$, de poly-γ-glutamate sous forme K$^+$, de poly-γ-glutamate sous forme NH$_4$$^+$, de poly-γ-glutamate sous forme Mg$^{++}$, de poly-γ-glutamate sous forme Ca$^{++}$, ou d'un mélange de deux de ces formes ou plus, réticulé avec un éther polyglycidylique de diglycérol, un éther polyglycidylique de polyglycérol, un éther polyglycidylique de sorbitol, un éther polyglycidylique de sorbitol polyéthoxylé, un éther polyglycidylique de polysorbitol, ou un mélange de deux composés tels ou plus, étant entendu que la quantité dudit agent hydratant représente de 0,005 % à 5 % du poids du produit cosmétique ou du produit de soin personnel.

2. Utilisation, conforme à la revendication 1, d'un hydrogel de poly-γ-glutamate et de poly(acide γ-glutamique) (γ-PGA, forme H), de poly-γ-glutamate sous forme Na$^+$, de poly-γ-glutamate sous forme K$^+$, de poly-γ-glutamate sous forme NH$_4$$^+$, de poly-γ-glutamate sous forme Mg$^{++}$, de poly-γ-glutamate sous forme Ca$^{++}$, ou d'un mélange de deux de ces formes ou plus, comme agent hydratant dans un produit cosmétique ou un produit de soin personnel.

3. Utilisation conforme à l'une des revendications précédentes, dans laquelle ledit hydrogel de poly-γ-glutamate présente un poids moléculaire de 15x10$^6$ à 200x10$^6$.

**4.** Utilisation conforme à l'une des revendications précédentes, dans laquelle lesdits poly(acide γ-glutamique) (γ-PGA, forme H), poly-γ-glutamate sous forme Na$^+$, poly-γ-glutamate sous forme K$^+$, poly-γ-glutamate sous forme NH$_4$$^+$, poly-γ-glutamate sous forme Mg$^{++}$, et poly-γ-glutamate sous forme Ca$^{++}$ présentent, indépendamment, un bas poids moléculaire de 100 000 à 500 000 ou un haut poids moléculaire de $10^6$ à $3 \times 10^6$.

**5.** Utilisation conforme à l'une des revendications précédentes, dans laquelle le produit est un produit de soin des mains, de soin du visage, de soin du corps, de soin des pieds, de soin de la tête, de soin des cheveux, de soin des ongles, ou de soin de la bouche.

**6.** Utilisation conforme à la revendication 1, dans laquelle la quantité dudit agent hydratant représente de 0,08 % à 1 % du poids dudit produit.

γ - poly - glutamic acid (or γ-PGA)

Repeating unit of M(I) γ - poly - (L) - glutamate
[M(I) γ - (L) - PGA]

Repeating unit of M(II)$_{1/2}$ γ -poly - (D) - glutamate

[M(II)$_{1/2}$ γ - (D) - PGA]

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61033107 A
- JP 10251402 A
- WO 2004007593 A
- WO 2004080433 A
- WO 2004039339 A
- JP 2003146830 A
- JP 2004210699 A
- JP 11343339 A
- JP 2002145723 A
- JP 1174397 A
- JP 2001354542 A
- JP 6322358 A

**Non-patent literature cited in the description**

- **MUZZARELLI, R. A. A.** Characteristic Properties of N-Carboxybutyl Chitosan. *Carbohydr. Polym.,* 1989, vol. 11, 307-320
- **DRAIZE, F.** Appraisel of the Safety of Chemicals in Food, Drugs and Cosmetics. *Pharmacolo,* 1948, vol. 93, 377-392